(19) Europäisches Patentamt
European Patent Office
Office européen des brevets

(11) **EP 3 566 729 B1**

(12) **EUROPEAN PATENT SPECIFICATION**

(45) Date of publication and mention
of the grant of the patent:
**24.03.2021 Bulletin 2021/12**

(51) Int Cl.:
***A61M 1/36*** *(2006.01)*

(21) Application number: **19173936.6**

(22) Date of filing: **10.05.2019**

(54) **HEMODIALYSIS MACHINE PROVIDED WITH A LIQUID FLOW MEASUREMENT SYSTEM AND RELATED METHOD**

HÄMODIALYSEMASCHINE MIT EINEM FLÜSSIGKEITS-DURCHFLUSS-MESSSYSTEM UND ZUGEHÖRIGES FUNKTIONSVERFAHREN

MACHINE D'HÉMODIALYSE MUNIE D'UN SYSTÈME DE MESURE DE FLUX DE LIQUIDE ET SON PROCÉDÉ DE FONCTIONNEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.05.2018 IT 201800005288**

(43) Date of publication of application:
**13.11.2019 Bulletin 2019/46**

(73) Proprietor: **Medica S.p.A.**
**41036 Medolla (IT)**

(72) Inventors:
• **BELLEI, Marco**
**41036 MEDOLLA (MO) (IT)**
• **BAGNOLI, Davide**
**41036 MEDOLLA (MO) (IT)**

(74) Representative: **Zamprogno, Bruno et al**
**STUDIO TORTA S.p.A.**
**Via Viotti, 9**
**10121 Torino (IT)**

(56) References cited:
**EP-A2- 1 343 576     WO-A1-2014/111908**
**GB-A- 1 385 595     US-A- 5 247 434**

**Description**

CROSS-REFERENCE TO RELATED APPLICATIONS

This patent application claims priority from Italian patent application no. 102018000005288 filed on 11/05/2018.

TECHNICAL FIELD

The present invention concerns a haemodialysis machine provided with a system for measuring the flowrate of a liquid and to the operating method thereof.
The present invention also concerns determining the flowrate differential between a first and a second liquid that flow through two respective ducts or channels of a haemodialysis machine so as to be able to advantageously determine the weight loss in a haemodialysis treatment performed with the haemodialysis machine.

BACKGROUND ART

As is known, haemodialysis machines generally comprise a haematic circuit that is provided with a filter basically consisting of a blood compartment and a dialyser compartment, mutually separated by a semi-permeable membrane and through which, in use, the blood to be treated and, respectively, a dialysing liquid, generally flowing in the opposite direction, can pass.
An arterial channel and a venous channel are connected with an inlet and with an outlet of the blood compartment, respectively, while an inflowing dialysis channel and an outflowing dialysis channel are connected with an inlet and an outlet of the dialyser compartment, respectively.
During the dialysis treatment, the unwanted particles contained in the blood migrate from the blood compartment to the dialyser compartment through the membrane due to the passage of a part of the liquid contained in the blood towards the dialyser compartment.
At the end of the dialysis treatment, a "weight loss" of the patient must be measured with extreme accuracy to avoid significant side effects, such as, collapse and cramps.
Some known systems/apparatus for measuring weight loss mounted in haemodialysis machines have the technical drawback of having footprints and production costs that have a non-negligible impact on the footprint and respectively the total cost of the haemodialysis machine.
For this purpose, the Applicant has designed a haemodialysis machine provided with a weight loss measurement system comprising a differential flowmeter operating substantially based on the operating principal of a thermal anemometer.
Said differential flowmeter is described, for example, in the European patent application EP 2 946 179 A1 filed by the Applicant, and basically comprises two channels adapted for respective haematic fluids to pass there through, and a pair of PTC (Positive Temperature Coefficient) heat-resistive sensors, each arranged in a respective channel. In use, each heat-resistive sensor is electrically heated so that its temperature reaches a value greater than a pre-set temperature of the fluid circulating in the relative channel. The flow of the fluid in the channel causes the dissipation of heat from the thermistor that determines a decrease in temperature and hence in the resistance of the sensor itself. The decrease in resistance caused by the fluids on the related heat-resistive sensors is subsequently processed by a differential electronic device that determines the differential flowrate of the fluids that flow through the two channels based on the resistance variation determined.
Although the differential flowmeter described above is particularly simple, inexpensive and accurate, the Applicant has carried out research in order to further increase the precision and sensitivity in determining the flowrate of a liquid in one or more channels and/or the differential flowrate of the liquids that pass through two channels Other similar systems are disclosed in documents WO2014111908 and US5247434.

DISCLOSURE OF INVENTION

The aim of the present invention is therefore to provide a solution that enables at least the objectives indicated above to be achieved.
This aim is achieved by the present invention as it relates to a haemodialysis machine (1) comprising a haematic circuit provided with a dialyser filter and one or more ducts that are connected to said dialyser filter and are adapted to allow respective liquids to pass there through, and a measurement system provided with a measurement circuit comprising one or more heat-resistive components associated with respective ducts, the haemodialysis machine being characterised in that said measurement system is configured to: supply an electric current having an alternation of first and second pre-set current values to said measurement circuit; said first current values being sized so that said heat-resistive components transmit a first quantity of negligible minimum heat towards the liquid that passes through the relative duct, said second current values being sized so that said heat-resistive components transmit a second quantity of heat significantly greater than said first quantity of heat, towards the liquid that passes through the relative duct; determine at least a first resistive value and a second resistive value indicative of a resistance variation on said at least one heat-resistive component caused by said alternation of first and respectively second current values; determine at least one differential resistive value indicative of said resistance variation based on the difference between said second resistive value and said first resistive value; determine the flowrate of a liquid passing through said duct and/or the flowrate differential of the liquid between the flowrate of said liquid and the flowrate of a liquid passing through another duct, based on said differential resistive value.

Preferably, the measurement system comprises a measuring apparatus provided with: current generator means connected to a heat-resistive component associated with a first duct for supplying the latter with said electric current, voltage measuring means for measuring a first voltage value of said heat-resistive component when the first current value passes through the latter and for measuring a second voltage value of said heat-resistive component, when said second current value passes through the latter, and calculation means configured to: calculate said first resistive value and said second resistive value based on said first voltage value and first current value and, respectively, on said second voltage value and second current value, calculate the differential resistive value based on the first and second resistive values, and calculate the flowrate of the liquid passing through the first duct based on said differential resistive value.

Preferably the measurement system comprises a second measuring apparatus provided with: current generator means connected to a heat-resistive component associated with a second duct for supplying the latter with said electric current, voltage measuring means for measuring a first voltage value of said heat-resistive component when the first current value passes through the latter and measuring a second voltage value of said heat-resistive component when said second current value passes through the latter, and calculation means configured so as to: determine said first resistive value and said second resistive value based on said first voltage value and said first current value and, respectively, on said second voltage value and second current value, determine the differential resistive value based on the first and second resistive values, and determine the flowrate of the liquid passing through the second duct based on the differential resistive value.

Preferably, the measurement system comprises a measuring apparatus provided with: a measurement branch comprising two heat-resistive components connected together in series and associated with a first duct and a second duct, respectively, current generator means connected to the measurement branch for supplying the latter with said electric current; voltage measurement means for measuring on each of said two heat-resistive components: a first voltage value and a second voltage value when said first and respectively second current values pass through the measurement branch; calculation means configured to: determine, for each of the two heat-resistive components, a differential resistive value based on the difference between the relative second and first resistive values.

Preferably, said calculation means are configured so as to determine the flowrate differential of the liquids passing through the first and the second ducts, based on said differential resistive values.

Preferably, the calculation means are configured so as to: calculate a first magnitude based on the difference between the two differential resistive values of the two ducts; determine the flowrate differential between the two ducts based on said first magnitude.

Preferably, the calculation means are configured so as to determine, for each of the two heat-resistive components, the flowrate of the liquid passing through the relative duct, based on the relative differential resistive value.

Preferably, the measurement system further comprises processing means configured so as to: receive a first flowrate of the liquid passing through the first duct, receive a second flowrate of the liquid passing through the second duct, determine the flowrate differential between the liquid passing through the first duct and the liquid passing through the second duct as a function of the first and second flowrates.

Preferably, the measurement circuit of said measuring apparatus comprises a Wheatstone bridge measurement circuit provided with a first heat-resistive component associated with a first duct, and a second heat-resistive component associated with a second duct.

Preferably, the measuring apparatus comprises current generator means for supplying said current to a first terminal of said bridge, voltage measuring means for measuring a first voltage value between two nodes of said bridge when the first current value passes through said first terminal and measuring a second voltage value between two nodes of said bridge when the second current value passes through said first terminal, and calculation means configured so as to: determine said first resistive value and said second resistive value based on said first voltage value and said first current value and respectively on said second voltage value and said second current value; determine the differential resistive value based on the first and second resistive values, and determine the flowrate differential between the liquid passing through the first duct and the liquid passing through the second duct, based on the differential resistive value.

Preferably, said electric current is a square wave current, in which said first and second current values are approximately constant.

The aim is also achieved by the present invention as it relates to an operating method of a haemodialysis machine comprising: a haematic circuit provided with a dialyser filter and one or more ducts that are connected to said dialyser filter and are adapted to allow respective liquids to pass there through, and a measurement circuit comprising one or more heat-resistive components associated with one or more ducts;

said method being characterised in that it comprises the steps of: supplying an electric current having alternation of first and second pre-set current values to said measurement circuit; said first current values being sized so that said heat-resistive components transmit a first quantity of negligible minimum heat towards the liquid that passes through the relative duct, said second current values being sized so that said heat-resistive components transmit a second quantity of heat significantly greater than said first quantity of heat to the liquid that passes through the relative duct; determining at least a first resistive value and a second resistive value indicative of a resistance variation on said at least one heat-resistive component caused by said alternation of first and second current values, respectively; determining at least one differential resistive value indicative of said resistance variation based on the difference between said second resistive value and said first resistive value; calculating the flowrate of a liquid passing through said duct and/or the flowrate differential of the latter with respect to a liquid passing through another duct based on said differential resistive value.

Preferably the measurement circuit comprises at least a heat-resistive component associated with a relative first duct for supplying the latter with said electric current; the method comprising the steps of: supplying, by means of current generator means, said electric current to said heat-resistive component, measuring, through voltage measuring means, a first voltage value of said heat-resistive component when the first current value passes through the latter, measuring a second voltage value of said heat-resistive component when said second current value passes through the latter, calculating said first resistive value and said second resistive value based on said first voltage value and said first current value and respectively on said second voltage value and said second current value; calculating a differential resistive value based on the first and second resistive values, and calculating the flowrate of the liquid passing through the first duct based on the differential resistive value.

Preferably said measurement circuit comprises a second measuring apparatus provided with: current generator means connected to a heat-resistive component associated with a second duct for supplying the latter with said electric current, voltage measuring means for measuring a first voltage value of said heat-resistive component when the first current value passes through the latter and measuring a second voltage value of said heat-resistive component when said second current value passes through the latter, the method comprising the steps of: calculating said first resistive value and said second resistive value based on said first voltage value and said first current value and, respectively, on said second voltage value and second current value, calculating the differential resistive value based on the first and second resistive values, calculating the flowrate of the liquid passing through the second duct based on the differential resistive value.

Preferably, the measurement system comprises a measuring apparatus provided with: a measurement branch comprising two heat-resistive components connected together in series and associated with a first and a second duct, respectively, current generator means connected to the measurement branch for supplying the latter with said electric current; voltage measuring means for measuring, on each of said two heat-resistive components: a first voltage value and a second voltage value when said first and second current values respectively pass through the measurement branch; the method comprising the step of determining, for each of the two heat-resistive components, a differential resistive value based on the difference between the relative second and first resistive values.

Preferably, the method further comprises the step of: determining the flowrate differential of the liquids passing through the first and the second ducts based on said differential resistive values.

Preferably, the method comprises: calculating a first magnitude based on the difference between the two differential resistive values of the two ducts; determining the flowrate differential between the two ducts based on said first magnitude.

Preferably, the method comprises the step of determining, for each of the two heat-resistive components, the flowrate of the liquid passing through the relative duct based on the relative differential resistive value.

BRIEF DESCRIPTION OF THE DRAWINGS

The present invention will now be described with reference to the accompanying drawings, which illustrate a non-limiting example of embodiment thereof, wherein:

- Fig. 1 schematically shows a haemodialysis machine produced according to the dictates of the present invention;
- Fig. 2 schematically shows a measurement system present in the haemodialysis machine shown in Fig. 1;
- Fig. 3 schematically shows a measurement circuit present in the measurement system shown in Fig. 1;
- Figs. 4, 5 and 6 show three graphs indicative of the trend of the three electrical magnitudes treated in the measurement system shown in Fig. 2;
- Fig. 7 schematically shows a haemodialysis machine produced based on a first variant of embodiment of the present invention;

- Fig. 8 schematically shows a measurement system present in the haemodialysis machine shown in Fig. 7;
- Fig. 9 schematically shows a measurement circuit present in the measurement system shown in Fig. 8;
- Fig. 10 schematically shows an alternative measurement system to the system shown in Fig. 8;
- Fig. 11 schematically shows an alternative measurement circuit to the measurement system shown in Fig. 9.

BEST MODE FOR CARRYING OUT THE INVENTION

The present invention will now be described in detail with reference to the accompanying figures to enable a person skilled in the art to produce and use it. Various modifications to the embodiments described will be immediately evident to those skilled in the art and the generic principles described can be applied to other embodiments and applications without thereby departing from the of the present invention, as defined in the appended claims. Therefore, the present invention must not be considered limited to the embodiments described and illustrated, but it must be granted the broadest scope of protection in conformity with the principles and characteristics described and claimed herein.

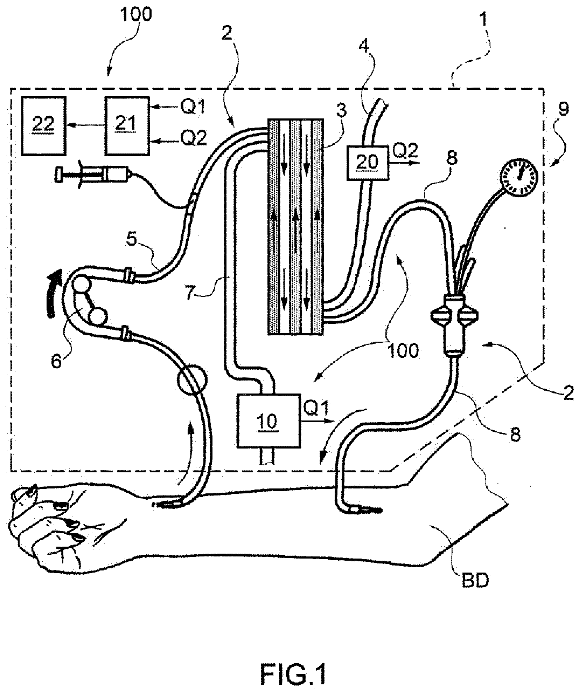

With reference to Fig. 1, the number 1 shows as a whole, in a simplified and schematic manner (with the aim of increasing understanding of the present invention), an example of a haemodialysis machine, that comprises a haematic circuit 2 provided with a dialyser filter 3, which can, for example, consist of a blood compartment and of a dialyser compartment, preferably separated from each other by a semi-permeable membrane through which, in use, the blood to be treated and, respectively, a dialysing liquid, preferably flowing in the opposite direction, can pass.

According to a possible example of embodiment shown in Fig. 1, the dialyser filter 3 can be supplied, on the one side (in a first inlet), with a dialysing liquid, i.e., a dialysate, through a duct 4, and on the other side (in a second inlet), with a haematic liquid, i.e., the arterial blood that flows through a duct 5, preferably as a result of the action of a pump 6, for example a peristaltic pump. The exchange of impurities between blood and dialysate takes place in the dialyser filter 3 according to a known process, which is not the subject matter of the present invention and shall not be further described, except to specify that in the example illustrated, the so-called "dirty" dialysate (containing impurities) flows out via the filter 3 flowing through a duct 7 (outlet), while the treated blood (filtered to remove the impurities) is reintroduced into the human body BD through a duct 8 (outlet) connected to the patient's vein.

The haemodialysis machine 1 can also be provided with a pressure measuring device 9, which, in the example illustrated, is arranged along the duct 8 to measure the blood pressure, preferably the venous pressure and detect the presence of air in the duct 5.

With reference to a preferred embodiment shown in Fig. 1, the haemodialysis machine 1 further comprises an electric/electronic measurement system 100 provided with at least one measuring apparatus 10 adapted to measure the flowrate of a liquid that passes through a duct of the haematic circuit 2.

In the example illustrated, and in the following description, purely by way of non-limiting example, the liquid in which the flowrate is measured by the measuring apparatus 10 corresponds to the dialysate circulating through the duct 7. However, it is understood that the measuring apparatus 10 produced according to the present invention can be associated with/connected to any duct present in the haemodialysis machine 1.

For example, with reference to the haemodialysis machine 1 shown in Fig. 1, the measuring apparatus 10 produced according to the present invention can be associated with the duct 4, or alternatively to the ducts 5 or 8 to measure the flowrate of the blood therein.

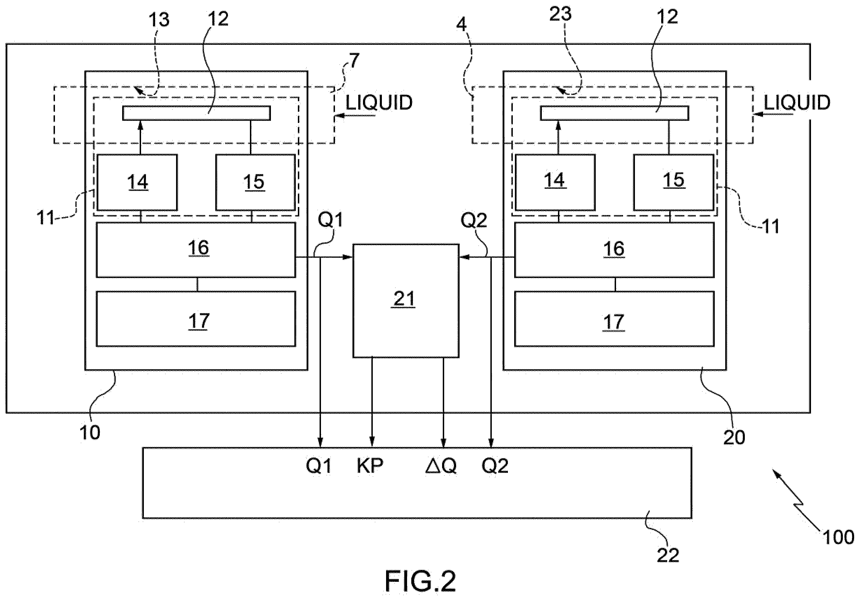

With reference to a preferred embodiment schematically illustrated in Fig. 2, the measuring apparatus 10 of the measurement system 100 basically comprises an electric/electronic measurement circuit 11, which is provided with at least an electric heat-resistive component 12. The electric heat-resistive component 12 can advantageously comprise, for example, a heat sensor, preferably ad anemometer, the electric heat-resistive (sensitive) component of which is used both as heat sink sensor and as temperature sensor. It is understood that the electric heat-resistive component 12 is used for temperature compensation of the fluid so that the flowrate is independent from the temperature of the fluid.

Preferably, the electric heat-resistive component 12 can comprise a PTC (Positive Temperature Coefficient) resistor or any similar electric/electronic thermistor component.

The electric heat-resistive component 12 can be associated with/coupled to/arranged in the duct 7 to be able to transmit and/or receive heat, directly or indirectly, to/from the liquid that passes through the duct 7.

With reference to the preferred embodiment schematically illustrated in Fig. 2, the electric heat-resistive component 12 can be arranged inside a measuring channel 13 hydraulically connected with the duct 7. In this way, the electric heat-resistive component 12 is advantageously wetted by the liquid being measured (in contact with the liquid) and varies its resistance as a function of the temperature and of the flowrate of this liquid. The measuring channel 13 can advantageously be included in the measuring device 10.

However, it is understood that the present invention can provide the measuring channel 13 separate from the measuring device 10. For example, the measuring channel 13 can correspond to a portion/stretch of the duct 7.

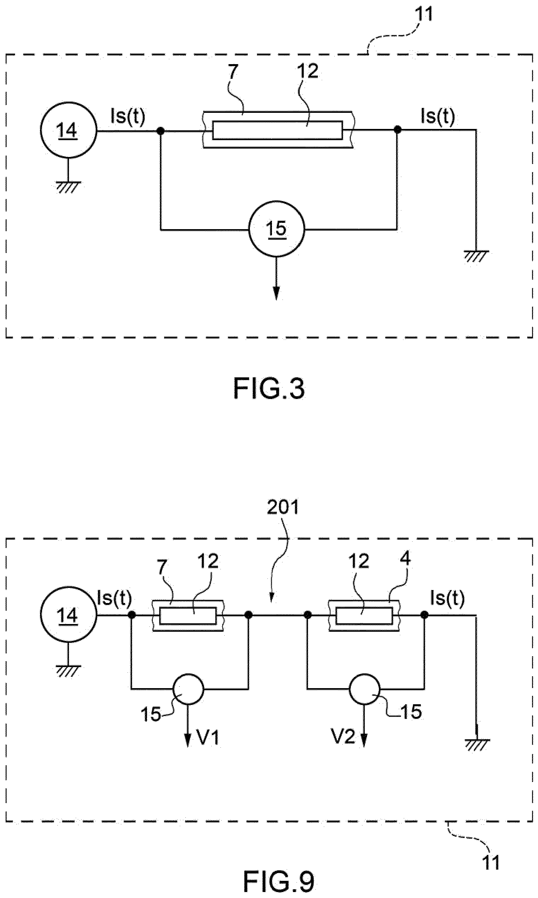

According to a preferred embodiment shown in Fig. 2 and schematized in Fig. 3, the measurement circuit 11 can preferably comprise a current generator device 14 adapted to supply a current Is(t) to the heat-resistive component 12.

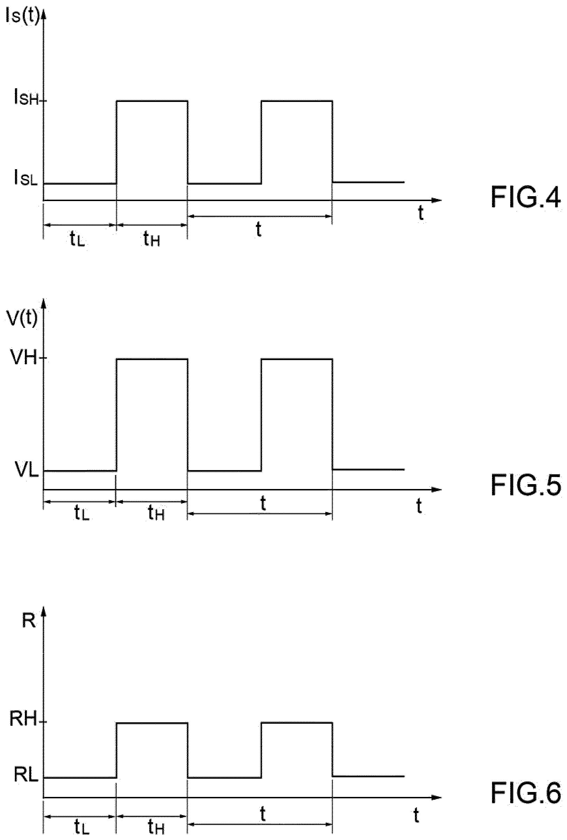

With reference to the example of Figs. 3 and 4, the current generator device 14 can generate an approximately square wave current Is(t). For each period t, the current Is(t) generated can alternate a first low current value ISL during a first half-period tL, and a high second current value ISH, greater than the first current value ISL, during the second half-period tH. Preferably, the two current values ISL and ISH can be approximately constant in the respective half-periods tL and tH.

The first current value ISL can be fixed (calibrated) to cause the transmission of a negligible quantity of heat by the heat-resistive component 12 towards the relative liquid. The term "negligible" means the transmission of a quantity of heat that does not cause a significant disturbance of the temperature of the liquid. In other words, the first current value ISL is such so as to cause heating through the Joule effect of the electric heat-resistive component 12, which however is extremely limited or negligible, i.e., is insufficient to cause a significant increase in the temperature of the liquid wets the heat-resistive component 12, a higher increase, for example lower than approximately 0.01°C.

The Applicant has found that a first current value ISL having a value comprised between around 0.5*0-3 Ampere and around 2*10-3 Ampere, preferably 1*10-3 Ampere does not significantly disturb the temperature of the liquid. With regard to the resistance of the heat-resistive component 12, in idle condition, it could be in the order of 100 Ohm. Preferably, the heat-resistive component 12 could advantageously be a platinum resistor.

Vice versa, the second current value ISH can be fixed (calibrated) to cause the transmission of a substantial quantity of heat by the heat-resistive component 12 towards the relative liquid. In other words, the second current value ISH is such so as to cause a (significant) pre-set heating through the Joule effect on the electric heat-resistive component 12 capable of causing an increase in the temperature of the liquid that wets the heat-resistive component 12, in the order of a few degrees, for example between around five degrees and around eight degrees.

The Applicant has found that the second current value ISH could advantageously be comprised between around $9*10^{-3}$ Ampere and around $15*10^{-3}$ Ampere, preferably $10*10^{-3}$ Ampere.

According to a preferred embodiment shown in Figs. 2 and 3, the measurement circuit 11 can further comprise a voltage measuring device 15, which is electrically connected to the heat-resistive component 12, for example at the terminals thereof. The voltage measuring device 15 is configured so as to measure the electrical voltages, designated by VL and VH, at the terminals of the resistive component 12 during the half-period t1L and respectively the half-period t1H (Fig. 5).

The measuring apparatus 10 can further comprise a calculation device 16 configured so as to implement the calculation operations described in detail below, for determining the flowrate of the liquid.

The calculation device 16 can be programmed/configured to:

Receive, from the voltage measuring device 15, the voltages VL and VH of electric the resistive component 12 measured during the half-period tL and respectively the half-period tH;
calculate a first resistive value RL indicative of the electrical resistance of the heat-resistive component 12 during the first half-period tL based on the voltage VL, i.e., RL=VL/ISL;
calculate a second resistive value RH indicative of the electrical resistance of the resistive component 12 during the second half-period tH based on the voltage VH, i.e., RH=VH/ISH;
calculate a differential resistive value Rd indicative of the variation of the resistance of the heat-resistive component 12 in response to the two current values ISL and ISH, wherein Rd=RH-RL; and determine the flowrate Q1 of the liquid as a function of the differential resistive value Rd.

The Applicant has found that calculation of the flowrate Q1 based on the differential resistive value Rd performed as described above, advantageously allows a decrease in the error introduced by the temperature of the liquid in calculation of the flowrate. In other words, calculation of the flowrate Q1 based on the differential resistive value Rd makes it possible to obtain a strong temperature compensation, i.e., to eliminate the dependency of the convective flow on the temperature of the fluid. The Applicant has in fact found that the following occurs in the measuring apparatus 10.

The resistance R of the heat-resistive component 12 in contact with a liquid varies as a function of different parameters, such as: the flowrate of the liquid Q1, the current Is(t) and the temperature of the liquid tF, i.e.:

$$a) \quad R=R(Q1, \; Is(t), tF)$$

During the supply of the first current value ISL, the Joule effect of the resistive component 12 is negligible and does not cause temperature variations tF in the liquid or, vice versa, the aforesaid resistive component 12 is substantially at the temperature of this liquid.

In this condition and within given error limits, it can thus be assumed that:

$$b)\ R=RL=Ro*(1+\alpha*tF)=Ro*(1+\alpha*(tF-To)))$$

wherein: Ro is the resistance at a reference temperature tF=To=0°C.

During the supply of the second current value ISH, the temperature tF of the fluid undergoes an increase tH=tF+At; therefore, the following mathematical relation is valid:

$$c)\ R=RH=Ro*(1+\alpha*tH)=Ro*(1+\alpha*(tF+\Delta t))$$

wherein $\Delta t$ is the temperature variation caused by the Joule effect $\Delta t=\Delta t(Is(t),Q1)$ and depends on the current supplied to the heat-resistive component 12 and on the flowrate Q1 of the liquid. By solving the relation c) the relation d) is obtained

$$d)\ R\ =\ Ro*(1+\ \alpha*(tF+\Delta t(Is(t),Q1))$$

Maintaining the second value ISH in the second half-period tH at an approximately constant value allows the following relation to be obtained:

$$e)\ RH=\ Ro*(1+\ \alpha*(tF+\Delta t(Q1))$$

The differential resistive value Rd can thus be calculated through the following relation:

$$f)\ Rd=RH-RL=\ Ro*(1+\ \alpha*(tF+\Delta t(Q1))-\ Ro*(1+\alpha*tF)$$

from which the following mathematical relation is advantageously obtained:

$$g)\ Rd=\ Ro*\ \alpha\ *\ \Delta t(Q1)$$

The relation g) is indicative of the fact that the differential resistive value Rd depends substantially on the flowrate Q1, as also $\Delta t$ depends on the flowrate Q1. Therefore, the Applicant has found that a direct relation exists between differential resistive value Rd and the flowrate Q1 that does not depend on the temperature of the liquid.

According to a possible embodiment, the measuring apparatus 10 can be provided with a memory device 17 that stores/contains, for example, one or more calculation tables. At least one stored calculation table can contain, for example, a plurality of differential resistive values Rd(i) and a plurality of flowrates Q1(i). Each differential resistive value Rd(i) of the calculation table can be associated with a respective flowrate Q1(i) value. The flowrates Q1(i) and the relative differential resistive values Rd(i) contained in the calculation table can advantageously be determined in a calibration/testing step of the measuring apparatus 10. For example, the calibration step used to determine the calculation table can provide for: supplying the square wave current Is(t) in the resistive circuit 12 according to the two pre-set current values ISL and ISH; imparting a plurality of pre-set flowrates Q1(i) of liquid passing through the duct 7; calculating for each flowrate Q1(i) the relative differential resistive value Rd(i) based on what has been described above; and storing the flowrates Q1(i) imparted and the relative differential resistive values Rd(i) calculated. The calculation table can be used by the calculation device 16 as "calculation function" to determine the flowrate Q1 of the liquid based on the differential resistive value Rd(i) calculated.

It is understood that, in use, the measuring apparatus 10 can be configured to continuously reiterate the operations described above so as to determine, instant by instant, the differential resistive value Rd(i) and, based on this, the flowrate Q1. In this way, the measuring apparatus 10 can determine the variation of the flowrate Q1 over time. In other words, the measuring apparatus 10 can be configured to repeat the following operations at pre-set measurement instants: sample the voltages VL and VH at the instant of measurement, calculate the resistances RL and RH based on the voltages VL and VH sampled, determine the differential resistive values Rd based on the difference between the resistances RL and RH calculated, and thus determine the flowrate Q1 in the measurement instant as a function of the differential resistive value Rd calculated.

With reference to Fig. 1, the measurement system 100 can also advantageously be adapted to determine the flowrate differential $\Delta Q$ between the flowrate Q1 between the liquid that passes through the duct 7 and the flowrate Q2 of the liquid that passes through another duct, such as the duct 4 in the haemodialysis machine 1 shown in Fig. 1. For this purpose, the measurement system 100 can be provided with another measuring apparatus, designated below with 20.

The measuring apparatus 20 can be functionally and structurally identical to the measuring apparatus 10 described above, the component parts of which will be designated with the same reference numbers that indicate corresponding parts of the measuring apparatus 10.

In the example shown in Figs. 1 and 2, the measuring apparatus 20 is associated with the duct 4, is configured to determine the flowrate Q2 of the relative dialysing liquid supplied, at inlet, to the dialyser filter 3. The measuring apparatus 20 is configured so as to calculate the flowrate Q2, carrying out the same operations described above implemented by the measuring apparatus 10 for calculations.

The measurement system 100 can be provided with an electronic processing device 21 configured so as to: receive, at inlet, the flowrates Q1 and Q2 determined by the measuring apparatus 10 and 20, and calculate the differential $\Delta Q$ between the flowrate Q1 of the outflowing dialysing liquid and the flowrate Q1 of the inflowing dialysing liquid, $\Delta Q = Q1-Q2$.

The electronic processing device 21 can also be configured to determine a value indicative of the weight loss KP based on the differential flowrate $\Delta Q$ between the dialysing liquids flowing, at inlet and outlet, of the dialyser filter 3. It is understood that the haemodialysis machine 1 can be provided with a user interface device 22, for example a display mounted in a control panel or the like, to notify the user of a series of information associated with the haemodialysis process. The information notified to the user can be, for example: the flowrates of the liquids Q1 and Q2 that pass through the relative ducts, the differential flowrate $\Delta Q$, and/or the weight loss Kp (total mass of liquids removed from the patient during the dialysis session).

According to this configuration, which provides for the use of two measuring apparatus 10 and 20 on two ducts 7 and 4 and the use of an electronic processing device 21 to determine the differential flowrate between the two ducts 7 and 4, the measurement system 100 advantageously performs the function of a differential flowmeter.

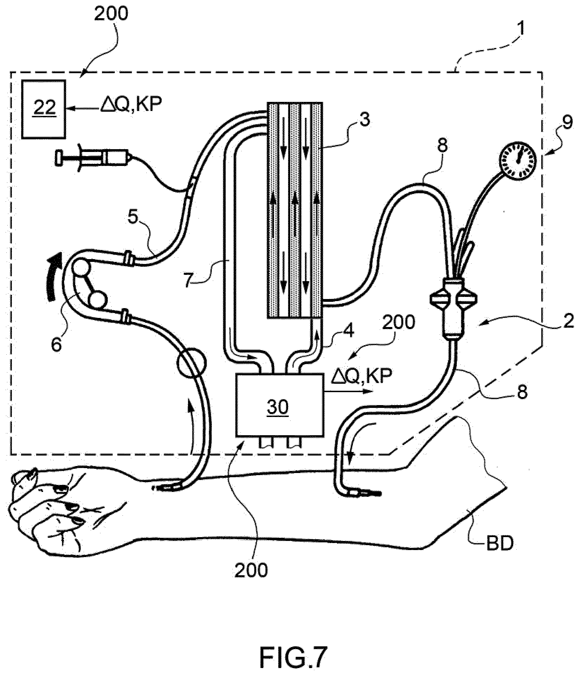

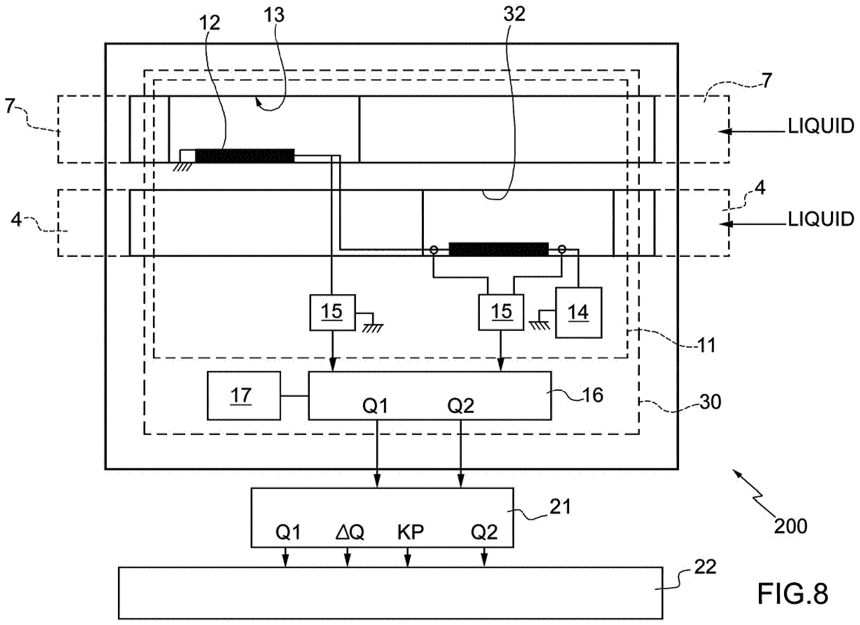

The embodiment illustrated in Fig. 7 relates to a measurement system 200, which is similar to the measurement system 100 shown in Fig. 1 and the component parts of which will be designated, where possible, with the same reference numbers that designate corresponding parts of the measurement system 100. With reference to Figs. 7, 8 and 9, the measurement system 200 comprises a measuring apparatus 30, which differs from the measuring apparatus 10 in that: the measurement circuit 11 comprises a measurement branch 201 comprising, in turn, two heat-resistive components 12 connected together in series and associated with two respective ducts. In the example illustrated, the heat-resistive components 12 are preferably associated with the duct 7 and respectively with the duct 4. The measuring apparatus 30 further comprises the current generator device 14 that is connected to the measurement branch 201 for supplying the electric current Is(t) to the latter.

The measurement circuit 11 of the measuring apparatus 30 further comprises two voltage measuring devices 15, which are respectively connected to the heat-resistive components 12 to measure the voltages V1 and V2 at the terminals of the latter.

The measuring apparatus 30 is configured to perform the following operations. The current generator 14 supplies the preferably square wave current Is(t) to the measurement branch 201; the voltage measuring devices 15 measure, during each first half-period tL, the voltages VL1 and VL2 present at the terminals of the two heat-resistive components 12 and, during each second half-period tH, the voltages VH1 and VH2 present at the terminals of the two heat-resistive components 12.

The calculation device 16 is configured to perform the following operations:

calculate a resistive value RL1 indicative of the electrical resistance of the first heat-resistive component 12 during the first half-period tL based on the voltage VL1, i.e., RL1=VL1/ISL;

calculate a resistive value RL2 indicative of the electrical resistance of the second heat-resistive component 12 during the first half-period tL based on the voltage VL2, i.e., RL2=VL2/ISL;

calculate a resistive value RH1 indicative of the electrical resistance of the first heat-resistive component 12 during the second half-period tH based on the voltage VH1, i.e., RH1=VH1/ISH;

calculate a resistive value RH2 indicative of the electrical resistance of the second heat-resistive component 12 during the second half-period tH based on the voltage VH2, i.e., RH2=VH2/ISH

calculate a differential resistive value Rd1 based on the difference between the resistive value RH1 and the resistive value RL1, i.e., Rd1=RH1-RL1; and

calculate a differential resistive value Rd2 based on the difference between the resistive value RH2 and the resistive value RL2, i.e., Rd2=RH2-RL2.

determine the flowrate Q1 of the liquid that passes through the first duct, preferably the duct 7, based on the differential resistive value Rd1 and

determine the flowrate Q2 of the liquid that passes through the second duct, preferably the duct 4, based on the differential resistive value Rd2.

It is understood that the flowrates Q1 and Q2 can be determined by the calculation device 16 through relative calculation functions F1(Rd1) and F2(Rd2). It is also understood that, in the same way as the measurement system 100 described above, the calculation functions F1(Rd1) and F2(Rd2) can be associated with respective calculation tables relative to the two ducts, preferably the ducts 4 and 7. The calculation tables can contain a plurality of differential resistive values Rd(i) and a plurality of flowrates Q(i). In the calculation tables, each differential resistive value Rd(i) can preferably be associated with a respective flowrate value Q(i). The flowrates Q(i) and the relative differential resistive values Rd(i) can advantageously be determined during the calibration/testing step of the measurement system 200.

In the measuring apparatus 30, the electronic processing device 21 can be configured so as to: receive the two flowrates Q1 and Q2 from the calculation device 16; calculate the differential $\Delta$Q between the flowrates Q1 and Q2 of the liquid passing through the two ducts 7 and 4. The electronic processing device 21 can be further configured to calculate the weight loss Kp based on the flowrates Q1 and Q2.

It is understood that the operation of the measuring apparatus 30 is not limited to calculation of the differential $\Delta$Q based on the two flowrates Q1 and Q2 calculated but can include other calculation methods.

For example, the measuring apparatus 30 could advantageously be configured to calculate the differential flowrate $\Delta$Q based directly on the differential resistive values Rd(i) hence without performing the intermediate calculation of the flowrates Q1 and Q2, with consequent decrease in errors in calculation of the differential flowrate $\Delta$Q. In this case, the calculation device 16 can be configured so as to:

calculate a resistive value RD indicative of the difference between the differential resistive values Rd1 and Rd2, preferably corresponding to the difference thereof, i.e.: RD=Rd1-Rd2,
and the electronic processing device 21 can be configured to:
calculate the differential flowrate $\Delta$Q based directly on the resistive value RD, through the following calculation function $\Delta$Q=FD(RD)=FD(Rd1-Rd2).

Also in this case, the calculation function FD can be defined, for example, through a calibration table. The calibration table can be stored in the storage device 17 of the measurement system 200 and contain a plurality of resistive values RD(i) and a plurality of flowrate differentials $\Delta$Q(i). For example, the calibration table can be structured so that each resistive value RD(i) can preferably be associated with a respective flowrate differential $\Delta$Q(i). It is understood that also in this case the electronic processing device 21 of the system 200 can calculate the weight loss Kp based on the flowrate differential $\Delta$Q(i).

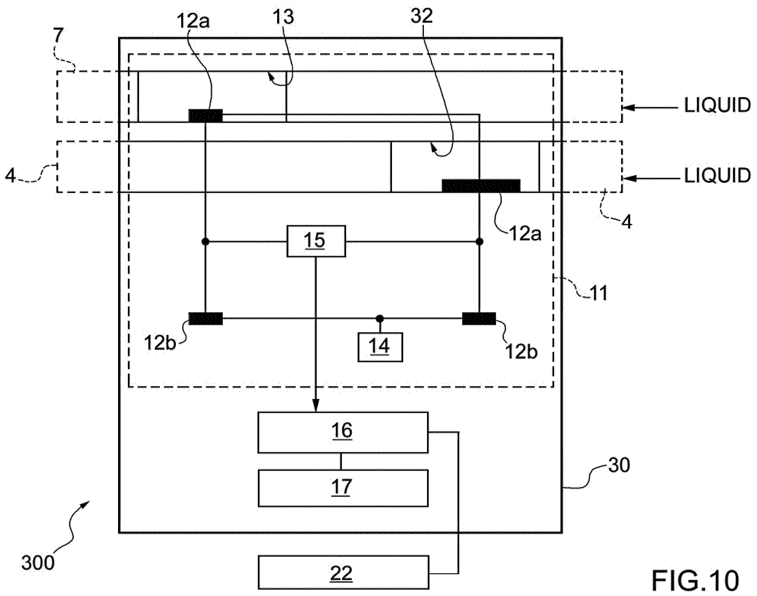

The embodiment illustrated in Fig. 10 relates to a measurement system 300, which is similar to the measurement system 100 shown in Fig. 1 and the component parts of which will be designated, where possible, with the same reference numbers that designate corresponding parts of the measurement system 100.

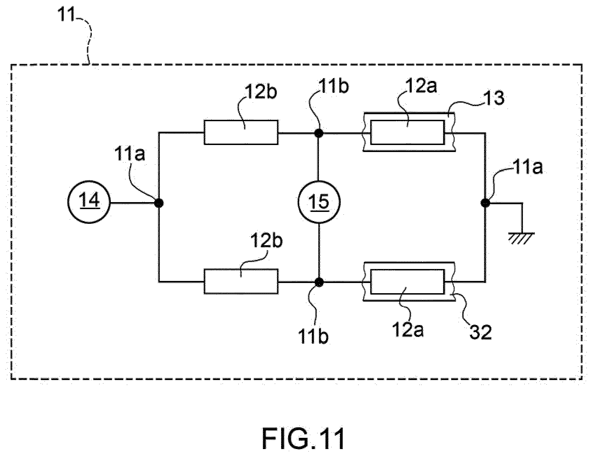

With reference to Figs. 10 and 11, the measuring apparatus 30 differs from the measuring apparatus 10 in that the relative measurement circuit 11 comprises a Wheatstone bridge measurement circuit preferably supplied by current.

According to a possible example of embodiment shown in Fig. 11, the Wheatstone bridge measurement circuit comprises a terminal 11a connected to the current generator device 14 to receive the current Is(t), a terminal 11a connected to a reference terminal GND, preferably a terminal placed at a ground potential, and two measurement nodes 11b between which the voltage measurement device 15 is connected. The Wheatstone bridge further comprises four resistive components arranged on four respective circuit branches connected respectively between a terminal 11a and a measurement node 11b. In the example illustrated, two resistive components 12a of the Wheatstone bridge comprise two respective PTC temperature sensor components, while the other two resistive components 12b comprise resistors having a pre-set (fixed) resistance. The two resistive components 12a are arranged inside respective measurement channels 13 and 32 associated with the ducts 4 and 7 so as to be wetted in use by the two liquids subject to the measurement and are thus adapted to vary the relative resistances as a function of the temperatures tf1 and tf2 and of the flowrates Q1 and Q2 of the liquids.

Instead, with regard to the voltage measurement device 15, it can be configured so as to determine a voltage indicative of a condition of unbalance of the bridge relative to a pre-set condition of balance of the bridge.

The measuring apparatus 30 of the measurement system 300 is configured to perform the following operations. The current generator 14 supplies the Wheatstone bridge, for example the node 11a, with the preferably square wave current Is(t) having in the half-periods tL and tH, the first ISL and respectively the second current value ISH (Fig. 3), and the voltage measurement device 15 measures the voltage VdL between the nodes 11b during each first half-period tL, and the voltage VdH present between the nodes 11b during each second half-period tH.

The measuring apparatus 30 of the measurement system 300 is further configured so that the calculation device 16 determines a first resistive value RdL associated with the first half-period tL. The calculation device 16 can determine the first resistive value RdL through the following mathematical relation:

$$i)\ RdL= VL/ISL$$

The measuring apparatus 30 is further configured so that the calculation device 16 determines a second resistive value RdH associated with the second half-period tH. The calculation device 16 determines the second resistive value RdH through the following mathematical relation:

$$l)\ RdH= VdH/ISH$$

The measuring apparatus 30 of the measurement system 300 is further configured so that the calculation device 16 determines the differential resistive value Rd based on the difference between the second resistive value RdH associated with the second half-period tH and the first resistive value RdL associated with the first half-period tL, i.e. Rd=RdH-RdL.

The measuring apparatus 30 of the measurement system 300 is further configured so that the calculation device 16 determines the differential ΔQ between the flowrate Q1 of the liquid that passes through the measuring channel 13 and the flowrate Q2 of the liquid that passes through the measuring channel 32 based on the differential resistive value Rd calculated.

Similarly to the measurement system 100, the measuring apparatus 30 of the system 300 can also be provided with a storage device 17 containing at least one calculation table that contains a plurality of differential resistive values Rd(i) and a plurality of flowrate differentials ΔQ(i). Preferably in the calculation table, each differential resistive value Rd(i) can be associated with a respective flowrate differential ΔQ(i). Also in this case, the numerical values contained in the table can advantageously be determined by performing calibrations/tests implemented on the measuring apparatus 30 which provide for: supplying the square wave current Is(t) having the first and second pre-set values ISL and ISH to the Wheatstone bridge, imparting the pre-set flowrates Q(i) of liquid in the two channels 13 and 32 according to pre-set flowrate differentials ΔQ(i), calculating for each flowrate differential ΔQ(i) the relative differential resistive value Rd(i), and storing the flowrate differential ΔQ(i) and the corresponding differential resistive value Rd(i) in the calibration table. The haemodialysis machine described above has various advantages.

Firstly, the measurement system describe above according to the different embodiments has high measurement precision, greater in particular, for example, in machines provided with CCA (Constant Current Anemometer) measurement systems in which both the temperature and the flowrate are measured.

In fact, Constant Current Anemometers supply a constant current to the measurement circuit, perform temperature measurement with a dedicated sensor, and calculate the flowrate based on the temperature measured. The present system differs from conventional Constant Current Anemometers as, besides supplying the circuit with a square wave current, it does not provide for direct measurement of the temperature to calculate the flowrate and thus eliminates from the latter the errors associated with temperature measurement. In other words, as the system described above does not measure the temperature directly, it is not subject to the relative measurement errors.

The measurement system, and in particular the temperature compensation of the measurement itself, due to: use of the same sensitive element used both to compensate the temperature of the fluid and to measure the flowrate of the latter; calculation of the flowrate compensated through the differential calculation of the resistance Rd without necessarily measuring/calculating the temperature of the liquid is in fact advantageously strong, as it greatly decreases the uncertainties introduced in the measurement above all due to a partial cancellation of systemic errors, both during calibration and measurement.

Moreover, the configurations described above on the one hand allow the use of chips of limited sizes in order to reduce the relative costs, and on the other allow the times required for calibration of the system to be halved, which obviously results in a reduction in costs for the performance thereof.

Finally, it is clear that modifications and variants can be made to the haemodialysis machine described above without departing from the scope of the present invention defined by the accompanying claims.

**Claims**

1.  A haemodialysis machine (1) comprising:

    a haematic circuit (2) provided with a dialyser filter (3) and one or more ducts that are connected to said dialyser filter (3) and are designed to allow respective liquids to pass there through, and
    a measurement system (100)(200)(300) provided with a measurement circuit (11) comprising one or more heat-resistive components (12) (12a) associated with respective ducts,

the machine for haemodialysis being **characterised in that** said measurement system (100)(200) is configured to:

supply said measurement circuit (11) with an electric current (Is(t)) having an alternation of first (ISL) and second (ISH) pre-set current values;

said first current values (ISL) being sized so that said heat-resistive components (12)(12a) transmit a first quantity of negligible minimum heat towards the liquid that passes through the relative duct,

said second current values (ISH) being sized so that said heat-resistive components (12)(12a) transmit a second quantity of heat significantly greater than said first quantity of heat, towards the liquid that passes through the relative duct;

determine at least a first resistive value (RL) (RdL) (Rd1) and a second resistive value (RH)(RdH)(Rd2) indicative of resistance variation on said at least one heat-resistive component (12) (12a) caused by said alternation of first (ISL) and respectively, second (ISH) current values;

determine at least one differential resistive value (Rd)(RD) indicative of said resistance variation based on the difference between said second resistive value (RH)(RdH)(Rd2) and said first resistive value (RL) (RdL) (Rd1) ;

determine the flowrate of a liquid (Q) passing through said duct and/or the flowrate differential ($\Delta$Q) of the liquid between the flowrate (Q1) of said liquid and the flowrate (Q2) of a liquid passing through another duct, based on said differential resistive value (Rd)(RD).

2. The machine according to claim 1, wherein said measurement system (100) comprises a measuring apparatus (10) provided with:

current generator means (14) connected to a heat-resistive component (12) associated with a first duct for supplying the latter with said electric current (Is(t)),

voltage measuring means (15) for measuring a first voltage value (VL) of said heat-resistive component (12) when the first current value (ISL) passes through the latter, and for measuring a second voltage value (VH) of said heat-resistive component (12), when said second current value (ISH) passes through the latter, and

calculation means (16) configured so as to:

calculate said first resistive value (RL) and said second resistive value (RH) based on said first voltage value (VL) and on said first current value (IL) and, respectively, on said second voltage value (VH) and on said second current value (ISH),

calculate the differential resistive value (Rd) based on the first (RL) and the second (RH) resistive values, and

calculate the flowrate (Q1) of the liquid passing through the first duct, based on said differential resistive value (Rd) .

3. The machine according to claim 2, wherein said measurement system (100) comprises a second measuring apparatus (20) provided with: current generator means (14) connected to a heat-resistive component (12) associated with a second duct for supplying said electric current (Is(t)) to the latter, voltage measuring means (15) for measuring a first voltage value (VL) of said heat-resistive component (12) when the first current value (ISL) passes through the latter (12), and measuring a second voltage value (VH) of said heat-resistive component (12) when said second current value (ISH) passes through the latter (12), and calculation means (16) configured so as to: determine said first resistive value (RL) and said second resistive value (RH) based on said first voltage value (VL) and first current value (ISL) and, respectively, said second voltage value (VH) and second current value (ISH), determine the differential resistive value (Rd) based on the first (RL) and second (H) resistive values, and determine the flowrate (Q2) of the liquid passing through the second duct based on the differential resistive value (Rd).

4. The machine according to claim 1, wherein said measurement system (200) comprises a measuring apparatus (30) provided with:

a measurement branch (201) comprising two heat-resistive components (12) connected together in series and associated with a first and, respectively, a second duct,

current generator means (14) connected to the measurement branch (201) for supplying said electric current (Is(t)) to the latter;

voltage measurement means (15) for measuring, on each of said two heat-resistive components (12): a first voltage value (VL1)(VL2) and a second voltage value (VL1)(VL2) when said first (ISL) and respectively second (ISH) current values pass through the measurement branch (201);

calculation means (16) configured so as to:
determine, for each of the two heat-resistive components (12), a differential resistive value (Rd1)(Rd2) based on the difference between the relative second (RH1)(RH2) and relative first (RL1)(RL2) resistive values.

5. The machine according to claim 4, wherein said calculation means (16) are configured so as to determine the flowrate differential ($\Delta$Q) of the liquids passing through the first and the second ducts based on said differential resistive values (Rd1)(Rd2).

6. The machine according to claim 4, wherein said calculation means (16) are configured so as to:

    calculate a first magnitude (RD) based on the difference between the two differential resistive values (Rd1)(Rd2) of the two ducts;
    determine the flowrate differential ($\Delta$Q) between the two ducts based on said first magnitude (RD).

7. The machine according to claim 4, wherein said calculation means (16) are configured so as to determine, for each of the two heat-resistive components (12), the flowrate of the liquid (Q1)(Q2) passing through the relative duct based on the relative differential resistive value (Rd1)(Rd2).

8. The machine according to claim 3 or 7, wherein said measurement system (100) further comprises processing means (21) configured so as to: receive a first flowrate (Q1) of the liquid passing through the first duct, receive a second flowrate (Q2) of the liquid passing through the second duct, determine the differential flowrate ($\Delta$Q) between the liquid passing through the first duct and the liquid passing through the second duct as a function of the first (Q1) and second (Q2) flowrates.

9. The machine according to claim 1, wherein said measurement circuit (11) of said measuring apparatus (30) comprises a Wheatstone bridge measurement circuit provided with a first heat-resistive component (12a) associated with a first duct, and a second heat-resistive component (12a) associated with a second duct.

10. An operating method for a haemodialysis machine (1) comprising: a haematic circuit (2) provided with a dialyser filter (3) and one or more ducts that are connected to said dialyser filter (3), are adapted to allow respective liquids to pass there through, and a measurement circuit (11) comprising one or more heat-resistive components (12)(12a) associated with one or more ducts;
said method being **characterised in that** it comprises the steps of:

    supplying said measurement circuit (11) with an electric current (Is(t)) having alternation of first (ISL) and second (ISH) pre-set current values; said first current values (ISL) being sized so that said heat-resistive components (12) (12a) transmit a first quantity of negligible minimum heat towards the liquid that passes through the relative duct, said second current values (ISH) being sized so that said heat-resistive components (12)(12a) transmit a second quantity of heat significantly greater than said first quantity of heat towards the liquid that passes through the relative duct;
    determining at least a first resistive value (RL) (RdL) (Rd1) and a second resistive value (RH)(RdH)(Rd2) indicative of a resistance variation on said at least one heat-resistive component (12) (12a) caused by said alternation of first (ISL) and respectively second (ISH) current values;
    determining at least one differential resistive value (Rd)(RD) indicative of said resistance variation based on the difference between said second resistive value (RH)(RdH)(Rd2) and said first resistive value (RL) (RdL) (Rd1) ;
    calculating the flowrate of a liquid (Q) passing through said duct and/or the flowrate differential ($\Delta$Q) of the latter in respect to a liquid passing through another duct based on said differential resistive value (Rd)(RD).

**Patentansprüche**

1. Hämodialysemaschine (1) mit:

    einem Blutkreislauf (2), der mit einem Dialysatorfilter (3) und einem oder mehreren Kanälen versehen ist, die mit dem Dialysatorfilter (3) verbunden sind und derart ausgebildet sind, dass entsprechenden Flüssigkeiten ermöglicht ist, durch sie hindurchzufließen, und
    einem Messsystem (100) (200) (300), das mit einer Messschaltung (11) versehen ist, die eine oder mehrere wärmebeständige Komponenten (12) (12a) aufweist, welche mit entsprechenden Kanälen verbunden sind, wobei die Maschine zur Hämodialyse **dadurch gekennzeichnet ist, dass** das Messsystem für Folgendes ausgebildet ist:

Versorgen der Messschaltung (11) mit einem elektrischen Strom (Is(t)), der zwischen ersten (ISL) und zweiten (ISH) voreingestellten Stromwerten wechselt;

wobei die ersten Stromwerte (ISL) so bemessen sind, dass die wärmebeständigen Komponenten (12) (12a) eine erste Menge an geringfügiger Mindestwärme an die Flüssigkeit abgeben, die durch den jeweiligen Kanal fließt,

wobei die zweiten Stromwerte (ISH) so bemessen sind, dass die wärmebeständigen Komponenten (12) (12a) eine zweite Menge an Wärme, die erheblich größer ist als die erste Menge an Wärme, an die Flüssigkeit abgeben, die durch den jeweiligen Kanal fließt;

Bestimmen mindestens eines erstes Widerstandswerts (RL) (RdL) (Rd1) und eines zweites Widerstandswerts (RH) (RdH) (Rd2), die eine Widerstandsänderung an der mindestens einen wärmebeständigen Komponente (12) (12a) angeben, die durch den Wechsel zwischen ersten (ISL) und zweiten (ISH) Stromwerten verursacht wird;

Bestimmen mindestens eines Differenzwiderstandswerts (Rd) (RD), der die Widerstandsänderung basierend auf der Differenz zwischen dem zweiten Widerstandswerts (RH) (RdH) (Rd2) und dem ersten Widerstandswert (RL) (RdL) (Rd1) angibt;

Bestimmen der Durchflussrate einer durch den Kanal fließenden Flüssigkeit (Q) und/oder der Durchflussratendifferenz (ΔQ) der Flüssigkeit zwischen der Durchflussrate (Q1) der Flüssigkeit und der Durchflussrate (Q2) einer durch einen anderen Kanal fließenden Flüssigkeit basierend auf dem Differenzwiderstandswert (Rd) (RD).

2. Maschine nach Anspruch 1, wobei das Messsystem (100) eine Messvorrichtung (10) aufweist, die mit Folgendem versehen ist:

Stromgeneratorvorrichtungen (14), die mit einer wärmebeständigen Komponente (12) verbunden sind, welche mit einem ersten Kanal verbunden ist, um die wärmebeständige Komponente (12) mit elektrischem Strom (Is(t)) zu versorgen,

Spannungsmessvorrichtungen (15) zum Messen eines ersten Spannungswerts (VL) der wärmebeständigen Komponente (12), wenn der erste Stromwert (ISL) durch letztere fließt, und zum Messen eines zweiten Spannungswerts (VH) der wärmebeständigen Komponente (12), wenn der zweite Stromwert (ISH) durch letztere fließt, und

Rechenvorrichtungen (16) die für Folgendes ausgebildet sind:

Berechnen des ersten Widerstandswerts (RL) und des zweiten Widerstandswerts (RH) basierend auf dem ersten Spannungswert (VL) und dem ersten Stromwert (ISL) bzw. auf dem zweiten Spannungswert (VH) und dem zweiten Stromwert (ISH),

Berechnen des Differenzwiderstandswerts (Rd) basierend auf dem ersten (RL) und dem zweiten (RH) Widerstandswert und Berechnen der Durchflussrate (Q1) der durch den ersten Kanal fließenden Flüssigkeit basierend auf dem Differenzwiderstandswert (Rd).

3. Maschine nach Anspruch 2, wobei das Messsystem (100) eine zweite Messvorrichtung (20) aufweist, die mit Folgendem versehen ist:

Stromgeneratorvorrichtungen (14), die mit einer wärmebeständigen Komponente (12) verbunden sind, welche mit einem zweiten Kanal verbunden ist, um die wärmebeständige Komponente (12) mit elektrischem Strom (Is(t)) zu versorgen,

Spannungsmessvorrichtungen (15) zum Messen eines ersten Spannungswerts (VL) der wärmebeständigen Komponente (12), wenn der erste Stromwert (ISL) durch letztere (12) fließt, und zum Messen eines zweiten Spannungswerts (VH) der wärmebeständigen Komponente (12), wenn der zweite Stromwert (ISH) durch letztere (12) fließt, und

Rechenvorrichtungen (16) die für Folgendes ausgebildet sind:

Bestimmen des ersten Widerstandswerts (RL) und des zweiten Widerstandswerts (RH) basierend auf dem ersten Spannungswert (VL) und dem ersten Stromwert (ISL) bzw. auf dem zweiten Spannungswert (VH) und dem zweiten Stromwert (ISH),

Bestimmen des Differenzwiderstandswerts (Rd) basierend auf dem ersten (RL) und dem zweiten (RH) Widerstandswert und Bestimmen der Durchflussrate (Q2) der durch den zweiten Kanal fließenden Flüssigkeit basierend auf dem Differenzwiderstandswert (Rd).

4. Maschine nach Anspruch 1, wobei das Messsystem (200) eine Messvorrichtung (30) aufweist, die mit Folgendem versehen ist:

einem Messzweig (201), der zwei wärmebeständige Komponenten (12) aufweist, die in Reihe geschaltet sind und mit einem ersten bzw. einem zweiten Kanal verbunden sind,
Stromgeneratorvorrichtungen (14), die mit dem Messzweig (201) verbunden sind, um letzteren mit elektrischem Strom (Is(t)) zu versorgen;
Spannungsmessvorrichtungen (15) zum Messen von Folgendem an jeder der zwei wärmbeständigen Komponenten (12): einem ersten Spannungswert (VL1) (VL2) und einem zweiten Spannungswert (VL1) (VL2), wenn die ersten (ISL) und die zweiten (ISH) Stromwerte durch den Messzweig (201) verlaufen;
Rechenvorrichtungen (16), die für Folgendes ausgebildet sind:
Bestimmen eines Differenzwiderstandswerts (Rd1) (Rd2) für jede der zwei wärmebeständigen Komponenten (12) basierend auf der Differenz zwischen dem jeweiligen zweiten (RH1) (RH2) und dem jeweiligen ersten (RL1) (RL2) Widerstandswert.

5. Maschine nach Anspruch 4, wobei die Rechenvorrichtungen (16) dazu ausgebildet sind, die Durchflussratendifferenz (ΔQ) der durch den ersten und den zweiten Kanal fließenden Flüssigkeiten basierend auf den Differenzwiderstandwerten (Rd1) (Rd2) zu bestimmen.

6. Maschine nach Anspruch 4, wobei die Rechenvorrichtungen (16) für Folgendes ausgebildet sind:

Berechnen einer ersten Größe (RD) basierend auf der Differenz zwischen den zwei Differenzwiderstandswerten (Rd1) (Rd2) der zwei Kanäle;
Bestimmen der Durchflussratendifferenz (ΔQ) zwischen den zwei Kanälen basierend auf der ersten Größe (RD).

7. Maschine nach Anspruch 4, wobei die Rechenvorrichtungen dazu ausgebildet sind, für jede der zwei wärmebeständigen Komponenten (12) die Durchflussrate der durch den jeweiligen Kanal fließenden Flüssigkeit (Q1) (Q1) basierend auf dem jeweiligen Differenzwiderstandswert (Rd1) (Rd2) zu bestimmen.

8. Maschine nach Anspruch 3 oder 7, wobei das Messsystem (100) ferner Verarbeitungsvorrichtungen (21) aufweist, die für Folgendes ausgebildet sind:

Empfangen einer ersten Durchflussrate (Q1) der durch den ersten Kanal fließenden Flüssigkeit,
Empfangen einer zweiten Durchflussrate (Q2) der durch den zweiten Kanal fließenden Flüssigkeit,
Bestimmen der Differenzdurchflussrate (ΔQ) zwischen der durch den ersten Kanal fließenden Flüssigkeit und der durch den zweiten Kanal fließenden Flüssigkeit in Abhängigkeit von der ersten (Q1) und der zweiten (Q2) Durchflussrate.

9. Maschine nach Anspruch 1, wobei die Messschaltung (11) der Messvorrichtung (30) eine Wheatstone-Brücken-Messschaltung aufweist, die mit einer ersten wärmebeständigen Komponente (12a) versehen ist, welche mit einem ersten Kanal verbunden ist, sowie mit einer zweiten wärmebeständigen Komponente (12a) versehen ist, welche mit einem zweiten Kanal verbunden ist.

10. Funktionsverfahren für eine Hämodialysemaschine (1) mit: einem Blutkreislauf (2), der mit einem Dialysatorfilter (3) und mit einem oder mehreren Kanälen versehen ist, die mit dem Dialysatorfilter (3) verbunden sind und dazu geeignet sind, entsprechenden Flüssigkeiten zu ermöglichen, durch sie hindurchzufließen, und einer Messschaltung (11), die eine oder mehrere wärmebeständige Komponenten (12) (12a) aufweist, die mit einem oder mehreren Kanälen verbunden sind;
wobei das Verfahren **dadurch gekennzeichnet ist, dass** es die folgenden Schritte umfasst:

Versorgen der Messschaltung (11) mit einem elektrischen Strom (Is(t)), der zwischen ersten (ISL) und zweiten (ISH) voreingestellten Stromwerten wechselt;
wobei die ersten Stromwerte (ISL) so bemessen sind, dass die wärmebeständigen Komponenten (12) (12a) eine erste Menge an geringfügiger Mindestwärme an die Flüssigkeit abgeben, die durch den jeweiligen Kanal fließt,

wobei die zweiten Stromwerte (ISH) so bemessen sind, dass die wärmebeständigen Komponenten (12) (12a) eine zweite Menge an Wärme, die erheblich größer ist als die erste Menge an Wärme, an die Flüssigkeit abgeben, die durch den jeweiligen Kanal fließt;

Bestimmen mindestens eines erstes Widerstandswerts (RL) (RdL) (Rd1) und eines zweites Widerstandswerts (RH) (RdH) (Rd2), die eine Widerstandsänderung an der mindestens einen wärmebeständigen Komponente (12) (12a) angeben, die durch den Wechsel zwischen ersten (ISL) und zweiten (ISH) Stromwerten verursacht wird;

Bestimmen mindestens eines Differenzwiderstandswerts (Rd) (RD), der die Widerstandsänderung basierend auf der Differenz zwischen dem zweiten Widerstandswerts (RH) (RdH) (Rd2) und dem ersten Widerstandswert (RL) (RdL) (Rd1) angibt;

Bestimmen der Durchflussrate einer durch den Kanal fließenden Flüssigkeit (Q) und/oder der Durchflussratendifferenz (ΔQ) der letzteren in Bezug auf eine durch einen anderen Kanal fließende Flüssigkeit basierend auf dem Differenzwiderstandswert (Rd) (RD).

**Revendications**

1. Machine d'hémodialyse (1) comprenant :

un circuit hématique (2) pourvu d'un filtre de dialyseur (3) et d'un ou plusieurs conduits qui sont connectés audit filtre de dialyseur (3) et qui sont conçus pour permettre le passage de liquides respectifs à travers ceux-ci, et un système de mesure (100) (200) (300) pourvu d'un circuit de mesure (11) comprenant un ou plusieurs composants résistants à la chaleur (12) (12a) associés à des conduits respectifs,

la machine pour hémodialyse étant **caractérisée en ce que** ledit système de mesure (100) (200) est configuré pour :

fournir audit circuit de mesure (11) un courant électrique (Is(t)) ayant une alternance de première (ISL) et deuxième (ISH) valeurs de courant prédéfinies ;

lesdites premières valeurs de courant (ISL) ayant une grandeur telle que lesdits composants résistants à la chaleur (12) (12a) transmettent une première quantité de chaleur minimale négligeable vers le liquide qui passe à travers le conduit relatif,

lesdites deuxièmes valeurs de courant (ISH) ayant une grandeur telle que lesdits composants résistants à la chaleur (12) (12a) transmettent une deuxième quantité de chaleur sensiblement supérieure à ladite première quantité de chaleur, vers le liquide qui passe à travers le conduit relatif ;

déterminer au moins une première valeur résistive (RL) (RdL) (Rd1) et une deuxième valeur résistive (RH) (RdH) (Rd2) indiquant une variation de résistance sur ledit au moins un composant résistant à la chaleur (12) (12a) causée respectivement par ladite alternance de première (ISL) et deuxième (ISH) valeurs de courant ;

déterminer au moins une valeur résistive différentielle (Rd) (RD) indiquant ladite variation de résistance sur la base de la différence entre ladite deuxième valeur résistive (RH) (RdH) (Rd2) et ladite première valeur résistive (RL) (RdL) (Rd1) ;

déterminer le débit d'un liquide (Q) passant à travers ledit conduit et/ou le différentiel de débit (ΔQ) du liquide entre le débit (Q1) dudit liquide et le débit (Q2) d'un liquide passant à travers un autre conduit, sur la base de ladite valeur résistive différentielle (Rd) (RD).

2. Machine selon la revendication 1, dans laquelle ledit système de mesure (100) comprend un appareil de mesure (10) pourvu :

de moyens générateurs de courant (14) connectés à un composant résistant à la chaleur (12) associé à un premier conduit pour fournir à ce dernier ledit courant électrique (Is(t)),

de moyens de mesure de tension (15) pour mesurer une première valeur de tension (VL) dudit composant résistant à la chaleur (12) lorsque la première valeur de courant (ISL) passe à travers ce dernier, et pour mesurer une deuxième valeur de tension (VH) dudit composant résistant à la chaleur (12), lorsque ladite deuxième valeur de courant (ISH) passe à travers ce dernier, et

de moyens de calcul (16) configurés de manière à :

calculer ladite première valeur résistive (RL) et ladite deuxième valeur résistive (RH) sur la base de ladite première valeur de tension (VL) et de ladite première valeur de courant (IL) et, respectivement, de ladite deuxième valeur de tension (VH) et de ladite deuxième valeur de courant (ISH),

calculer la valeur résistive différentielle (Rd) sur la base des première (RL) et deuxième (RH) valeurs résistives, et

calculer le débit (Q1) du liquide passant à travers le premier conduit, sur la base de ladite valeur résistive différentielle (Rd).

3.  Machine selon la revendication 2, dans laquelle ledit système de mesure (100) comprend un deuxième appareil de mesure (20) pourvu :

de moyens générateurs de courant (14) connectés à un composant résistant à la chaleur (12) associé à un deuxième conduit pour fournir ledit courant électrique (Is(t)) à ce dernier, de moyens de mesure de tension (15) pour mesurer une première valeur de tension (VL) dudit composant résistant à la chaleur (12) lorsque la première valeur de courant (ISL) passe à travers ce dernier (12), et mesurer une deuxième valeur de tension (VH) dudit composant résistant à la chaleur (12) lorsque ladite deuxième valeur de courant (ISH) passe à travers ce dernier (12), et de moyens de calcul (16) configurés de manière à : déterminer ladite première valeur résistive (RL) et ladite deuxième valeur résistive (RH) sur la base de ladite première valeur de tension (VL) et de la première valeur de courant (ISL) et, respectivement, de ladite deuxième valeur de tension (VH) et de la deuxième valeur de courant (ISH), déterminer la valeur résistive différentielle (Rd) sur la base des première (RL) et deuxième (H) valeurs résistives, et déterminer le débit (Q2) du liquide passant à travers le deuxième conduit sur la base de la valeur résistive différentielle (Rd).

4.  Machine selon la revendication 1, dans laquelle ledit système de mesure (200) comprend un appareil de mesure (30) pourvu :

d'une branche de mesure (201) comprenant deux composants résistants à la chaleur (12) connectés entre eux en série et associés respectivement à un premier et un deuxième conduit,

de moyens générateurs de courant (14) connectés à la branche de mesure (201) pour fournir ledit courant électrique (Is(t)) à cette dernière ;

de moyens de mesure de tension (15) pour mesurer, sur chacun desdits deux composants résistants à la chaleur (12) : une première valeur de tension (VL1) (VL2) et une deuxième valeur de tension (VL1) (VL2) lorsque lesdites première (ISL) et deuxième (ISH) valeurs de courant passent respectivement à travers la branche de mesure (201) ;

de moyens de calcul (16) configurés de manière à :

déterminer, pour chacun des deux composants résistants à la chaleur (12), une valeur résistive différentielle (Rd1) (Rd2) sur la base de la différence entre la deuxième valeur résistive relative (RH1)(RH2) et la première valeur résistive relative (RL1)(RL2).

5.  Machine selon la revendication 4, dans laquelle lesdits moyens de calcul (16) sont configurés de manière à déterminer le différentiel de débit (ΔQ) des liquides passant à travers les premier et deuxième conduits sur la base desdites valeurs résistives différentielles (Rd1) (Rd2) .

6.  Machine selon la revendication 4, dans laquelle lesdits moyens de calcul (16) sont configurés de manière à :

calculer une première amplitude (RD) sur la base de la différence entre les deux valeurs résistives différentielles (Rd1) (Rd2) des deux conduits ;

déterminer le différentiel de débit (ΔQ) entre les deux conduits sur la base de ladite première amplitude (RD) .

7.  Machine selon la revendication 4, dans laquelle lesdits moyens de calcul (16) sont configurés de manière à déterminer, pour chacun des deux composants résistants à la chaleur (12), le débit du liquide (Q1) (Q2) passant à travers le conduit relatif sur la base de la valeur résistive différentielle (Rd1) (Rd2) relative.

8.  Machine selon la revendication 3 ou 7, dans laquelle ledit système de mesure (100) comprend en outre des moyens de traitement (21) configurés de manière à : recevoir un premier débit (Q1) du liquide passant à travers le premier conduit, recevoir un deuxième débit (Q2) du liquide passant à travers le deuxième conduit, déterminer le débit différentiel (ΔQ) entre le liquide passant à travers le premier conduit et le liquide passant à travers le deuxième conduit en fonction des premier (Q1) et deuxième (Q2) débits.

9.  Machine selon la revendication 1, dans laquelle ledit circuit de mesure (11) dudit appareil de mesure (30) comprend un circuit de mesure en pont de Wheatstone pourvu d'un premier composant résistant à la chaleur (12a) associé à un premier conduit, et d'un deuxième composant résistant à la chaleur (12a) associé à un deuxième conduit.

10. Procédé de fonctionnement pour une machine d'hémodialyse (1) comprenant : un circuit hématique (2) pourvu d'un filtre de dialyseur (3) et d'un ou plusieurs conduits qui sont connectés audit filtre de dialyseur (3), qui sont adaptés pour permettre le passage de liquides respectifs à travers ceux-ci, et un circuit de mesure (11) comprenant un ou plusieurs composants résistants à la chaleur (12) (12a) associés à un ou plusieurs conduits ;
ledit procédé étant **caractérisé en ce qu'**il comprend les étapes consistant à :

fournir audit circuit de mesure (11) un courant électrique (Is(t)) ayant une alternance de première (ISL) et deuxième (ISH) valeurs de courant prédéfinies ; lesdites premières valeurs de courant (ISL) ayant une grandeur telle que lesdits composants résistants à la chaleur (12) (12a) transmettent une première quantité de chaleur minimale négligeable vers le liquide qui passe à travers le conduit relatif, lesdites deuxièmes valeurs de courant (ISH) ayant une grandeur telle que lesdits composants résistants à la chaleur (12) (12a) transmettent une deuxième quantité de chaleur sensiblement supérieure à ladite première quantité de chaleur vers le liquide qui passe à travers le conduit relatif ;
déterminer au moins une première valeur résistive (RL) (RdL) (Rd1) et une deuxième valeur résistive (RH) (RdH) (Rd2) indiquant une variation de résistance sur ledit au moins un composant résistant à la chaleur (12) (12a) causée respectivement par ladite alternance de première (ISL) et deuxième (ISH) valeurs de courant ;
déterminer au moins une valeur résistive différentielle (Rd) (RD) indiquant ladite variation de résistance sur la base de la différence entre ladite deuxième valeur résistive (RH) (RdH) (Rd2) et ladite première valeur résistive (RL) (RdL) (Rd1) ;
calculer le débit d'un liquide (Q) passant à travers ledit conduit et/ou le différentiel de débit (ΔQ) de ce dernier par rapport à un liquide passant à travers un autre conduit sur la base de ladite valeur résistive différentielle (Rd) (RD).

FIG.1

FIG.2

EP 3 566 729 B1

FIG.4

FIG.5

FIG.6

FIG.7

FIG.8

EP 3 566 729 B1

FIG.3

FIG.9

23

FIG.10

EP 3 566 729 B1

FIG.11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IT 102018000005288
- EP 2946179 A1
- WO 2014111908 A
- US 5247434 A